# EUROPEAN PATENT SPECIFICATION

(11) **EP 0 446 300 B1**
(45) Date of publication and mention of the grant of the patent: **05.06.1996**
(21) Application number: 90901237.9
(22) Date of filing: 28.11.1989
(51) Int. Cl.: A61L 27/00, A61F 2/32, C08L 23/06

(54) **ULTRAHIGH MOLECULAR WEIGHT LINEAR POLYETHYLENE, ARTICLES AND PROCESSES OF MANUFACTURE**
LINEARES POLYÄTHYLEN VON ULTRAHOHEM MOLEKULARGEWICHT, GEGENSTÄNDE UND VERFAHREN ZU DEREN HERSTELLUNG
POLYETHYLENE LINEAIRE DE POIDS MOLECULAIRE TRES ELEVE, OBJETS ET PROCEDES DE FABRICATION

(30) Priority: 02.12.1988 US 278913; 22.12.1988 US 288577; 24.10.1989 US 426916
(43) Date of publication of application: 18.09.1991
(73) Proprietor: E.I. DU PONT DE NEMOURS AND COMPANY, Wilmington Delaware 19898 (US)
(72) Inventor: HOWARD, Edward, George, Jr., Hockessin, DE 19707 (US)
(74) Representative: Jones, Alan John
(86) International application number: US8905261
(87) International publication number: WO9006139

(56) References cited:
- Polymer, volume 22, January 1981, IPC Business Press, (London, GB), S.K. Bhateja: "Unaxial tensile creep behaviour of ultra high molecular weight linear polyethylene", pages 23-28, see page 28, conclusions
- Journal of Polymer Science : Part A2, Polymer Physics, volume 7, 1969, John Wiley & Sons, (New York, US) T.D. Davidson et al.: "Extended-chain Crystals. II. Crystallization of polyethylene under elevated pressure", pages 2051-2059 - see page 2052, lines 32-38

## Description

### BACKGROUND OF THE INVENTION

### 1. Field of the Invention

This invention relates to a novel ultrahigh molecular weight linear polyethylene (UHMWLPE). This novel UHMWLPE, in the form of a shaped article, exhibits in various embodiments a unique combination of properties making the material useful as a bearing surface, in general, but particularly useful as a prosthetic hip joint cup and as other prosthetic shapes for replacement of other joints of the human body.

### 2. Description of the Prior Art

In U.S. Patent No. 3,944,536 (March 1976), Lupton et al describe UHMWPE in the form of a fabricated article exhibiting an elastic modulus of 340,000 to 500,000 psi, a tensile impact strength of 140 to 600 ft lb/in², a density of 0.95 to 0.98 g/cc at 25°C, a crystalline melting point of 142 to 148°C (as measured by differential thermal analysis) and a unique crystalline form characterized by the absence of fold spacings of 50-2000 Angstrom units (Å) and the presence of crystal spacings of about 10,000 Å. The critical feature of the process of producing this UHMWPE is disclosed to involve inducing crystallization of the molten polymer above 150°C by rapidly increasing the applied pressure from an initial level of 1 to 1000 atmospheres to a second level of 2000 to 7000 atmospheres and then cooling rapidly while maintaining a pressure sufficient to maintain the polyethylene in the solid phase until the temperature is below the crystalline melting point of the polyethylene at atmospheric pressure.

In Kunstuffe German Plastics 77 (1987) pp. 617-622, in an article entitled "Ultrahigh Molecular Polyethylene for Replacement Joints", Eyrer et al. point out that the service life of joint replacements made of UHMWPE is limited. Analysis of the damage to over 250 explanted hip cups and tibial plateaus revealed a changed property profile which they explained by post-crystallization resulting from oxidative chain decomposition. They suggested optimizing the processing of polyethylene under higher pressure and higher temperature to increase the degree of crystallinity. The Eyrer et al. product displays a creep of above 5% at a compression of 1000 psi (6.9 N/mm²) for 24 hours at 37°C.

One of the most remarkable advances in the medical field in recent years is the development of prosthetic joints, particularly the load bearing hip. The crippled and sometimes bed ridden elderly can walk again. The key to this development is UHMWPE because, not only does it have the necessary impact strength, but it initiates no adverse blood reactions. But at present, these prosthetic joints are limited to the older, less active segment of the population because the polymer tends to creep under the pressure that a younger more active person might develop while involved in recreation or employment. The creep would cause the loss of the close tolerance required between the plastic socket and the polished metal ball attached to the femur. These changes in dimensions disturb the distribution of walking forces which in turn accelerates more creep and wear. Eventually the increased pain requires a traumatic revision operation. One objective of this invention is to provide UHMWPE prosthetic joints with improved creep resistance hence removing some of the age restriction existing on the present polyethylene joints.

### SUMMARY OF THE INVENTION

The object of this invention is to provide a tough UHMWLPE composition and article with creep resistance that is less than 1% (as measured at a temperature of 23 ± 1°C and a relative humidity of 50 ± 2% for 24 hours under a comparison of 1000 psi (6895 kPa)) and that maintains excellent tensile and flexural properties.

Specifically, the product of this invention is a composition exhibiting an elastic or flexural modulus of 250,000-500,000 psi (1724-3448 MPa), a tensile stress at yield of 3500-4500 psi (24-31 MPa), a tensile stress at break of 4000-9000 psi (28-62 MPa), a tensile modulus of 300,000-700,000 psi (2069-4827 MPa), preferably 300,000-600,000 psi (2069-4137 MPa), an elongation of 200-500%, a notched Izod impact resistance of 12-25 ft. lb. per in. (6.4-13.3 J/cm) of notch, a creep at a compression of 1000 psi of less than 1% after 24 hours at a temperature of 23°C and a relative humidity of 50%, the polyethylene having a molecular weight of 1,000,000-10,000,000 (the molecular chain length between folds being greater than 3500Å), a single crystalline melting point of greater than 144°C (as measured by differential scanning calorimetry) the reduction in said melting point upon reheating being greater than 11°C above that of the starting polymer and an infrared crystallinity index of at least about 0.45, preferably at least 0.5. The composition may be formed into an article.

The process for obtaining the article of this invention involves six (6) important steps:
1. forming, by milling or casting or the like the article from UHMWLPE having a molecular weight of 400,000-10,000,000, preferably at least 1,000,000 and most preferably at least 6,000,000;
2. placing the article in a pressure vessel (e.g. an autoclave) substantially filled with a fluid that is inert to the article, preferably water;
3. heating the vessel to a temperature of at least 190°C, preferably 200°C-230°C, and raising the pressure in the vessel, usually by adding additional water, to at least 2800 atmospheres (ATM) (284 MPa), preferably at least 3000 ATM (304 MPa);
   Alternately, the article, water, and reactor are heated to about 200-230°C, the pressure is applied and the system held at this temperature while polymer slowly loses heat of crystallization and solidifies.
   Also, the pressure can be applied at any temperature during the heating cycle if the temperature exceeds about 200°C.
4. maintaining the temperature and pressure substantially as selected in step 3 for at least 0.5 hour, preferably at least one hour;
5. thereafter, cooling by reducing the temperature to a temperature at least below about 160°C-170°C preferably to 160°C or below, most preferably below 140°C, while maintaining a pressure of at least 2800 ATM (284 MPa) preferably at least 3000 ATM (304 MPa), at a slow rate, the rate of cooling being such that temperature gradients in the shaped article are substantially avoided. The polymer must be cooled slowly at the high pressure until it is fully crystallized. At 3000 ATM (304 MPa) pressure, the crystallization temperature of UHMWLPE of over one million molecular weight is in the range of 170°C-190°C. The pressurized vessel should be cooled slowly to insure that the temperature of the polymer is not significantly above the vessel temperature, particularly if the pressure vessel construction does not permit means for measuring the temperature of the polymer itself.
6. cooling and releasing the pressure on the shaped article in a manner such that any remelting of the article is prevented. This is accomplished by cooling at least to a temperature below the atmospheric pressure melting point, i.e., about 130°C-135°C, preferably below 120°C, most preferably below 100°C, and releasing the pressure to reduce it, from at least 2800 ATM (284 MPa) to approximately 1 ATM (101 kPa), either sequentially or simultaneously. It should be understood that it is necessary to cool the polymer to a temperature below its melting point at any particular pressure to ensure that none of the polymer melts as the pressure is reduced since lowering the pressure lowers the melting point.

As an optional seventh step, it is advisable to shave the surface of the article, i.e. remove approximately the outer 2 millimeters that might contain any fluid-affected polymer.

A very important step is the fifth step, i.e. cooling in a manner that limits severe temperature gradients in the article. For example, for a 1 inch X 6 inch (2.54 X 15.2 cm) rod, a cooling rate of approximately 10°C per hour is usually necessary. Although cooling rates no greater than 10°C per hour are preferred, cooling rates as high as about 60°C per hour have been used to provide the product of this invention. However, these latter rates require careful control in order to limit temperature gradients during cooling. Cooling rapidly, as taught in the prior art, will not provide the article of this invention. This invention is particularly useful for manufacturing shaped articles where temperature gradients pose a problem during the cooling step, i.e., where the article's cross-sectional dimensions are at least 1 inch x at least 1 inch (2.54 x 2.54 cm), usually for joints at least 1 inch x at least 2 inches (2.54 x 5.08 cm). Specifically, the importance of this step and of this invention is manifest in producing articles having as its smallest dimension 0.2 inch (5 mm), i.e., at least 0.2 inch (5 mm) in thickness. It has been found that in such articles, the temperature gradients must still be controlled by the process of this invention in order to obtain the product of this invention.

Alternately, for steps 2 and 3, the pressure vessel can be used more efficiently by preheating polymer outside the reactor because heating polyethylene is a very slow process due to its high heat of fusion and its thermal insulation property. It can be heated in an oven, preferably in an inert atmosphere to prevent oxidation, particularly when the tempereature is above 160°C. Because the UHMW polyethylenes do not flow when melted, they can be handled while hot and transferred to the preheated pressure vessel without deforming.

Product resulting from this modified process also is excellent for orthopedic replacement and has certain superior characteristics. Values demonstrating these superior, enhanced qualities are shown in Example 11.

In addition to utility in the field of orthopedic replacement, the products prove useful in other applications also requiring the special properties of the products. Not only shaped articles are of interest, but also films and fibers as well as other "downstream" forms and unshaped granular forms of the product will prove useful. Film formed of the product of Example 5 is exemplified in Example 13. These examples are illustrative only, and other forms, shaped and unshaped, of the composition are contemplated within the scope of the invention. Therefore, "article" shall include both shaped articles and unshaped articles.

In a process more recently developed for manufacturing the product of this invention, the fluid used in the pressure vessel could be a gas that does not affect the UHMWLPE adversely. Specifically, the shaped article is formed from commercially available UHMWLPE. In the second step, the article is placed in a pressure vessel containing the fluid, in this case argon. However, it is necessary in the case where argon is used, to protect the UHMWLPE from any entry of the gas into the polymer by surrounding the article with a thin stainless steel or similar metal can as described in U.S-A-5037928 It should be understood that other gaseous fluids may be used in place of argon. So long as the gas does not adversely affect or is prevented from adversely affecting the polymer and is not affected by the temperatures and pressure used in the process, the gas may be used.

In the next step, a pressure of at least 2800 ATM (284 MPa) is applied and the vessel is heated to about 220°C for about 6 hours. Thereafter, the temperatur is "ramped" down at a rate no greater than about 10°C per hour to about 160°C while maintaining the pressure above 2800 ATM (284 MPa). The temperature is then "ramped" down at a maximum rate to 50°C while maintaining the high pressure, after which the pressure is released.

A further embodiment of the invention involves the following important steps:
1. forming by milling a casting or the like the article from UHMWPE having a molecular weight of 400,000-10,000,000, preferably at least 1,000,000;
2. subjecting said article to a temperature of 190-340°C, preferably 320-340°C, for at least 0.5°C hour, preferably at least one hour, in an inert atmosphere; and
3. cooling the article non-precipitously to a temperature of about 130°C or below. As in the other embodiments, the rate of cooling is such that temperature gradients in the shaped article are substantially avoided. Again, the rapid cooling sought in the prior art will not provide the article of this embodiment. The product of this non-pressurized embodiment is particularly useful for manufacturing shaped articles where temperature gradients pose a problem during the cooling step, i.e., where the article's cross-sectional dimensions are at least 1 inch x at least 1 inch (2.54 x 2.54 cm), usually for joints at least 1 inch x at least 2 inches (2.54 x 5.08 cm). Specifically, the importance of this step and of this invention is manifest in producing articles having as its smallest dimension 0.2 inch (5 mm), i.e., at least 0.2 inch (5 mm) in thickness.

For purposes of this invention, ultrahigh molecular weight linear polyethylene (UHMWLPE) is defined as a linear polyethylene having an estimated weight-average molecular weight of 400,000 to 10,000,000, usually 1,000,000 to 10,000,000 as defined by a melt index (ASTMD-1238) of essentially zero and a reduced specific viscosity (RSV) greater than 8, preferably 25-30. The relationships of RSV to intrinsic viscosity and to molecular weight are those developed by R. Chaing as presented by P. S. Francis et al. in J. Polymer Science, 31, 453 (1958).

It is envisaged that the modifications of adding a preliminary step heating the product to 320-340°C will also provide superior characteristics to the product described by Li.

The improved properties of the products of this invention in its various embodiments are reflected in a tensile modulus of at least 300 kpsi (2069 MPa), a flex modulus of at least 250 kpsi (1724 MPa), ultimate tensile strength greater than 4000 psi (28 MPa), yield strength greater than 3500 psi (24 MPa) and an elongation at break no greater than 500%.

A suitable polyethylene is one wherein the flexural modulus is 250-650 kpsi (1724-4482 MPa), the tensile stress at yield is 3.5-5.4 kpsi (24-27 MPa), the tensile stress at break is 4-6 kpsi (27.6-41.4 MPa), the tensile modulus is 300-700 kpsi (2069-4827 MPa), the elongation at break is 200-600%, the notched IZOD impact resistance is 12-25 ft. lb. per in. (6.4-13.3 J/cm) of notch and creep at a compression of 1 kpsi (6.9 MPa) is less than 1% after 24 hours at a temperature of 23°C and a relative humidity of 50%. Preferably the tensile modulus is 300-650 kpsi (2069-4482 MPa) and the infrared crystallinity is at least 0.5.

This polyethylene may be made by a process consisting essentially of the following steps:
(a) forming an article of an ultrahigh molecular weight linear polyethylene having a molecular weight of 400,000 to 10,000,000;
(b) subjecting said article to a preliminary heat treatment at a temperature of 320-340°C, in an inert atmosphere for at least 0.5 hours; and preferably continuing for at least 3 hours at a temperature of at least 320°C;
(c) subjecting said article to a fluid under pressure of at least 2800 atm (284 MPa) and a temperature of at least 190°C for at least 0.5 hours;
(d) reducing the temperature to 160-170°C or lower while maintaining the pressure at at least 284 MPa, the rate of reduction in temperature being such that temperature gradients in the shaped article are substantially avoided; and
(e) cooling to a temperature of about 130°C or lower and reducing the pressure to approximately 101 kPa in a manner such that remelting of said article is prevented.

A very important property of the product of this invention is its creep resistance. For this invention is its creep resistance. For prosthetic devices, e.g. knee, hip, elbow joints, etc., any substantial creep can be devastating in the loss of the benefits of extremely expensive surgery. Thus, the shaped articles of this invention display as little as a 0.5% loss in thickness when subjected to a compression pressure of 1000 psi (6.9 MPa) for 24 hours at a temperature of 23°C and a relative humidity of 50% in accordance with ASTM D-621.

Also according to this invention an improved folded chain ultrahigh molecular weight linear polyethylene has a molecular weight of at least 400,000, and exhibiting a flexural modulus of 150-300 kpsi (1034-2069 MPa), a tensile stress at yield of 3.5-4.0 kpsi (24-28 MPa), a tensile stress at break of 4-6 kpsi (28-41 MPa), a tensile modulus of 150-300 kpsi, a notched Izod impact resistance of 15-25 ft. lb. per in. (6.4-13.3 J/cm) of notch, an elongation at break of 200-800%, a creep at a compression of 1 kpsi (6.9 MPa) of less than 2% after 24 hours at a temperature of 23°C and a relative humidity of 50%, and an infrared crystallinity index of at least about 0.35.

For some applications, still lower creep, higher stiffness, higher elongation, and particularly higher tensile strengths at yield are necessary. These can be introduced by the enhancing process if the polyethylene is first thermally heated at 320-340°C for thirty minutes or longer. The process depends on heating the polymer as near as possible to, without reaching, its decomposition point. The hot polymer should be cooled slowly because very rapid cooling, such as immersion in cold water, causes internal voids to form. This is caused by the combination of large volume changes polyethylene undergoes when melting (about 30%) and its poor heat conductivity. A convenient method is to allow the polymer to cool wrapped in insulation. The hot polyethylene can be put directly into the hot pressure vessel or it can be cooled and reheated to the normal 200°C in the pressure vessel.

The improved properties of the product of this part of the invention are reflected in tensile modulus of at least 350 kpsi (2413 MPa), a tensile strength at yield of 3500-5400 psi (24-37 MPa), and a creep value of less than 0.6%.

In addition, the thermally heated polymer, still in the folded chain form used to make this superior form of this invention, in itself has improved elongation, crystallinity, and impact resistance over the starting material, but it is not equivalent to the enhanced form of polyethylene.

Perhaps the most characteristic property of the product of this invention is its infrared crystallinity index (IRCI). This property, which provides a reasonably accurate reflection of the crystallinity of this material, is in a range never before attained with any UHMW polyethylene materials. To determine this index, samples are first obtained by microforming thin sections. Heat and pressure should be avoided during preparation of the samples. ICRI is the ratio of the band at 1894 reciprocal centimeters (cm⁻¹) to the band at 1305 reciprocal centimeters (cm⁻¹). Since the band at 1894 cm⁻¹ is attributed to the crystalline nature of the material and the band at 1305 cm⁻¹ is attributed to its amorphous nature, ICRI increases as the crystallinity increases. The product of this invention displays an IRCI of at least about 0.45. In fact, values of 0.73 and higher have been obtained. On the other hand, IRCI values for prior known UHMWLPE's seldom reach above 0.3.

The invention will be more clearly understood by referring to the drawings and examples, which follow. In the drawings, Figure 1 is a schematic diagram of the equipment used in the process for forming the product of the invention using argon gas; and Figure 2 is a schematic diagram of the equipment used in the hydrostatic process.

In the examples, most of the properties are measured using standard ASTM tests.

All of the physical measurements were carried out under constant humidity (50% relative humidity) and temperature (23°C) conditions.

Tensile modulus, ultimate tensile strength, yield strength and elongation were measured according to ASTM D-638 with the following modifications:
- samples machined into shape without lubricating fluid
- type I tensile bar
- cross head speed = 0.2"/min (5 mm/min) for tensile modulus
   2.0"/min (51 mm/min) for tensile stress and elongation.

Creep resistance was measured in accordance with ASTM D-621 with the following modifications:
- samples machined into cylinders or cubes without the use of lubricating fluids
- samples measured 0.5" x 0.5" x 0.5" (1.27 x 1.27 x 1.27 cm)
Flexural properties were measured according to ASTM D-790 with the following modifications:
- samples machined into shape without the use of lubricating fluids
- typical flex bar measures 0.125" (3.2 mm) thick x 0.5" (12.7 mm) width x 5" (127 mm) length
- span or gage is 2.0". (51 mm) (This was determined by a span/depth ratio of 16/1.)
- cross head speed = 0.05"/min (0.1 mm/min) (calculated based on span).

Impact resistance was measured using the notched Izod test given in ASTM D-256 with the following modifications:
- samples machined into shape without the use of lubricating fluid
- type A or notched IZOD
- specimen size is 0.5" x 2.5" (12.7 x 63.5 mm)
- 0.4" (10 mm) from bottom of vertex to opposite side
- 1.25" (32 mm) impacted end (from end of bar to vertex of notch)
- the notch should be the specified angle of 22.5 degrees.

It should be appreciated that in all embodiments of the invention the step of forming the article by milling, casting, or the like from UHMWLPE may be performed as the first step in the process (i.e., before heating or preheating) or as the last step in the process (i.e., after the cooling step).

The following non-limiting examples, including the improved and superior embodiments, illustrate the basic principles and unique advantages of the present invention. Various changes and modifications may be made without departing from the spirit and scope of the present invention.

### EXAMPLE 1

The material used in this example is American Hoechst Hostalen 415 GUR ultrahigh molecular weight polyethylene. It was obtained in the form of bars, 3" (76 mm) in diameter and up to 5' long (1.5 m) in length. The material will be referred to as UHMWLPE. The molecular weight was over 1,000,000.

One or more pieces of the UHMWLPE 11 were placed into stainless steel, seamless, 48" (1.2 m) long cylinders or sleeves 12. The thickness of the stainless steel was 1/8" (3.2 mm). The bottom of the cylinders was closed by welding a stainless steel cap 13 onto the bottom of the cylinder. The top of the cylinder was partially closed by welding on a modified cap 14 which contained a vacuum port 15. The cylinder was then evacuated using a vacuum pump and sealed by crimping the port to form a can that surrounds the piece of UHMWLPE completely. The sealed cylinder was then placed in a containment vessel 16 large enough to hold 15 cylinders. The containment vessel 16 was then placed into a hot isostatic pressing (HIP) unit 17 with molybdenum heating units 18. Thermocouples were added to monitor the temperature of the cylinders.

The basic function of the HIP process is to uniformly heat a load while applying pressure uniformly to all surfaces. The pressure medium used in this case was argon. The UHMWPE is protected from contact with the argon by the stainless steel cans.

The process conditions were:
1. Apply pressure to 39,000 psi (269 MPa).
2. Heat to 220°C.
3. Hold for 6 hours at 220°C and a minimum pressure of 41,000 psi (27 atmos.)(283 MPa).
4. Ramp temperature down at a rate no faster than 10°C per hour to 160°C. Pressure is maintained above 41,000 psi (283 MPa) during this time.
5. Ramp temperature down at maximum rate to 50°C while maintaining the pressure above 41,000 psi (283 MPa).
6. Below 50°C, pressure may be let down and the cycle ended.

The UHMWPE rods were then removed from the sleeves and parts were fabricated for physical testing. It is noted that the material produced exhibits much higher tensile modulus, flex modulus, melting point, density and creep resistance than the starting material (Control A).

| Material | DSC Melting Point (°C) | Density (grams/cc) | IRCI |
|---|---|---|---|
| Control | 137.0-140.7°C | .93-.94 | 0.24 |
| Example 1 | 148.0-152.0°C | .947 | ≧ 0.45 |

| ASTM D638 | Control A | Example 1 | |
|---|---|---|---|
| Flex Modulus kpsi (MPa) | 165 (1138) | 291 (2006) | |
| Tensile Modulus kpsi (MPa) | 185 (1276) | 315 (2172) | |
| Ultimate Tensile kpsi (MPa) | 4500 (31028) | 4688 (32324) | |

| ASTM D638 | | | |
|---|---|---|---|
| Yield kpsi (MPa) | 3476 (23967) | 4082 (28145) | |
| Elongation (break) % | 262 | 227 | |
| [values are averages of 5 tests] | | | |

| ASTM D621 Creep Test | | |
|---|---|---|
| Load | | |
| 500 psi (3448 kPa) | .5 | .3 % deformation |
| 1000 psi (6895 kPa) | 1.6 | .7 |
| 2000 psi (13790) kPa) | 5.9 | 2.4 |

Additional evidence of the products' distinctiveness is found in data produced by small angle X-ray testing. A truly characteristic small-angle X-ray scattering plot of desmeared intensity (by the method of P. W. Schmidt, Acta Cryst., 13, 480 (1960) and Acta Cryst., 19, 938 (1965)) (I x (2 theta) squared) versus scattering angle (2 theta) for the material of the invention exhibits two distinct scattering peaks associated with crystal long-spacings in the range of 480 angstroms (at 2 theta = .184 degrees) and 4610 angstroms (at 2 theta = .0192 degrees). The presence of the sharp diffraction peak at the lower angle is indicative of an extended polymer chain conformation (with a lamellar thickness greater than 2000 angstroms) whereas the more diffuse higher-angle peak corresponds to a lamellar thickness characteristic of conventional folded chain PE. This provides clear evidence for the presence of two scattering peaks in the subject invention material which correspond to lamellar thicknesses both above and below 2000 angstroms. By comparison, the previously patented extended chain polyethylene of Lupton et al., was reported to exhibit a complete absence of any detectable small angle X-ray scattering in the range of 50 to 2000 angstroms. Consequently this work demonstrates that the subject invention material is morphologically distinguishable from Lupton et al.

### EXAMPLE 2

The material used in this example is an ultrahigh molecular weight polyethylene obtained from Jet Plastics, Inc.

A rod 21 measuring 6" x 1 1/8" (152 x 28.6 mm) was placed in the cavity 22 of a stainless steel, seamless, cylindrical reactor 23. The cavity 22 had a diameter of 1.35" (34 mm) and was about 9" (229 mm) long.

Water was fed into the cavity 22 at the entry port 24 through the use of a high pressure water pump 25 powered by compressed air. Simultaneously, the reactor was heated by electrical heaters 26 surrounding the reactor.

In the first step, the rod 21 was heated to a temperature of 220°C under a hydrostatic pressure of 2000 ATM (203 MPa). The pressure was raised to 3000 ATM (304 MPa) while the temperature was maintained at 220°C for 2 hours. The temperature was permitted to fall to 209°C over another 2 hour period, and then to about 182°C in 4 hours. Finally, the rod was cooled to 49°C by subjecting the reactor 23 to compressed air from the blower 27 over a period of one hour and the pressure released.

The rod was removed from the reactor and the surface was shaved. The product, a sample taken substantially from the center of the rod, displayed a DSC melting point of 154.5°C, and, on reheating, a DSC melting point of 140°C.

The material, when subjected to a compression pressure of 1000 psi for 24 hours at a temperature of 23°C and a relative humidity of 50% in accordance with ASTM D-621, deformed only 0.4%.

The other properties of the product were:

| | |
|---|---|
| flexural modulus | - over 250 kpsi (1724 MPa) |
| tensile modulus | - over 300 kpsi (2069 MPa) |
| tensile stress (yield) | - over 3500 psi (24132 MPa) |
| tensile stress (break) | - over 4000 psi (27580 MPa) |
| elongation (break) | - less than 500%. |

Its infrared crystallinity index was over 0.5.

The hydrostatic process described in Example 2 is the best mode for preparing the product of this invention. This process has important advantages. The pressure transfer liquid, water, is non-toxic and inexpensive. The hydraulic pressure is applied equally in all directions resulting in a substantially homogeneous product. This compares to processes shown in the prior art where hydraulic pressure is applied by a piston or a ram. In these latter cases, the high shrinkage polymer tends to solidify along the heat-escaping walls making it difficult for the pistons to advance and still apply the pressure uniformly. The result is a heterogeneous product.

It should be understood that although water is the preferred liquid fluid to use in the process, other liquids, with the same criteria as mentioned for gases, are also useful. Thus, methanol, ethanol, glycerin, glycols, etc. in addition to various aqueous solutions may be used.

The salt selected for an aqueous solution may be one that imparts a desirable property to the surface of the shaped article.

### EXAMPLE 3

This experiment was carried out in a manner similar to Example 2 except that the pressure in the first step was 3000 ATM. (304 MPa) The material was maintained at 220°C under 3000 ATM (304 MPa) for 4 hours. The temperature was allowed to fall to 190°C over an 8-hour period. After which, it was cooled to 100°C in 1 hour.

Samples were taken from 1/8" (3.2 mm) inside both ends of the rod and had melting points of 150.8°C and 153.2°C. When reheated, the melting points were 135.5°C and 138°C, respectively.

The infrared crystallinity index was 0.791; and the creep, when measured in accordance with ASTM D-621, was less than 1%. These measurements were obtained on a sample taken from the center of the rod.

### EXAMPLE 4

The experiment was also carried out in a manner similar to Example 2 except for the following changes in the heating/cooling cycle:
Heat at 211°C and 3000 ATM (304 MPa) and maintain for 1 hour;
Cool to 200°C in 1 hour at 3000 ATM (304 MPa);
Cool to 180°C over 5 hours at 3000 ATM (304 MPa) (cooling rate 200→180°C, 4°/hour); and
Cool to 33°C in 1 hour and 3 minutes.

The product from inside both ends melted at 150°C and on reheating, at 135.5°C. The product, when tested in accordance with ASTM D-621 displayed a creep of less than 1%. Its infrared crystallinity index was 0.652.

### EXAMPLE 5

A reactor with the general configuration shown in Figure 2 having an internal diameter of 4" (102 mm) and being 22" long (559 mm), was charged with a 3 1/8" x 18 1/16" (79 x 459 mm) rod of UHMWPE (made from polymer from American Hoechst, Hostalen GUR 415). The closed vessel was evacuated, filled with water, and heated to 232°C at which point the pressure was increased to 3000 ATM (304 MPa) with the water pump. This pressure was maintained until the end of the experiment. The reactor was held between 210 and 230°C for 3 hours, cooled over 1 hour to 200°C, cooled to 175°C in 5 hours (5°/hour) and then cooled to 80°C in 7 1/2 hours.

The resulting product rod was still in a cylindrical form with very little warpage. It measured 3 1/8" x 17 15/16" (79 x 456 mm). End pieces, 1/2" (12.7 mm) thick, were cut off each end of the rod revealing a uniform white color. Samples taken from the center of the rod on these cuts gave melting points of 152.9°C (201 J/g) and 152.1°C (207 J/g) when heated at 20°C/minute. When reheated, the melting points were 137.5°C.

A six inch section of the rod was sawn into 3/16" (5 mm) thick shapes for physical tests, then carefully milled to remove saw marks to 1/8" (3 mm) thickness. The resulting polymer had the following properties:

| | |
|---|---|
| IZOD | 18.7 ft. lb./in. (3.27 N/mm) of notch |
| Flexural Modulus | 298.9 kpsi (2061 MPa) |

| Tensile Properties | |
|---|---|
| Stress at yield | 4190 psi (28.9 MPa) |
| Stress (max.) (at break) | 5430 psi (37.4 MPa) |
| Elongation (at break) | 280% |
| Modulus | 378.3 kpsi (2608 MPa) |
| Creep Test, 1000 psi (6.9 MPa) | 0.6% |

All tests at room temperature.

The crystallinity index (IRCI) was 0.528.

### EXAMPLE 6

In this example, the product was prepared with an exceedingly smooth surface.

A polished brass disk, about 1 1/2" (38 mm) diameter, 1/4" (6 mm) thick was pressed at 160°C against a UHMW polyethylene plug. The combination was cooled under pressure and sealed in a heat shrinkable Teflon FEP™ tube. The polyethylene was converted in a hydrostatic system by this procedure in the vessel used in Example 5.

The heating cooling cycle was as follows:
3000 ATM (304 MPa) and 210°C in 1 hour;
3000 ATM (304 MPa) 210°C to 200°C in 1 hour;
3000 ATM (304 MPa) 200°C to 178°C in 6 hours, 45 minutes; and
3000 ATM (304 MPa) 178°C to 23°C in 2 hours, 20 minutes.

The polyethylene did shrink so that it had a smaller diameter than the disk, but the polymer stuck to the surface. When forced apart, the surface was extremely smooth.

This technique is important in preparing complicated surfaces where smoothness is extremely important, such as on bearing surfaces such as medical prosthesis for knee and hip joints, or bearings for motor shafts, etc. Machine cutting polymers always leaves very small ridges.

### EXAMPLE 7

The reactor of Figure 2, internal diameter 4" by 22" (10 by 56 mm) long was charged with a 3" x 18" (8 x 46 mm) rod of American Hoescht, Hostalen GUR 415 ultrahigh molecular weight polyethylene, water, and a nominal pressure of 100 psi (690 kPa). The system was heated to 170°C to 176°C and held there for 1 hour, then the pressure was raised to 3000 ATM (304 MPa). The temperature was maintained at 179°C-174°C for 3 hours, during which time the polyethylene crystallized. The reactor was cooled to 79°C in 1.7 hours.

Two samples were taken; one from the center of the rod and another 1/2 inch (12.7 mm) from the outer surface of the rod. The melting points, as measured by DSC, were 150.9°C and 150.4°C, respectively, and upon reheating, 136.6°C and 137/3°C. Thus, the increases in melting points were 14.3°C and 12.7°C, respectively. The infrared crystallinity index was 0.5.

### EXAMPLE 8

This example shows that the polymer can be cooled at a rate as high as 34.5°C per hour in the critical cooling step (step 5) if proper precautions are taken to limit temperature gradients.

A one inch rod of UHMWLPE from Jet Plastics, Inc. was used. It was placed in the pressure vessel with water and subjected to the following treatments:
3000 ATM (304 MPa) and 220°C for 2 hours;
3000 ATM, (304 MPa) cool to 200°C in 50 minutes;
3000 ATM (304 MPa), cool to 177°C in 40 minutes;
3000 ATM (304 MPa), cool to 35°C in one hour.

A test sample taken one-half inch from the end of the rod and in the center displayed a DSC melting point of 153.8°C, and, on reheating a DSC melting point of 139.7°C.

The material, when subjected to a compression pressure of 1000 psi (6895 kPa for 24 hours at 23°C and a relative humidity of 50% in accordance with ASTM D-621 deformed 0.5%.

### EXAMPLE 9

### Superior Enhanced UHMW Polyethylene prepared by Preheating Polymer to 325°C.

A 3-1/16" x 15" (31 x 381 mm) rod of UHMW polyethylene (Hoechst GUR415, fabricated by PolyHi) was heated to 325°C in an atmosphere of N₂ for six hours. The hot rod was quickly placed in a pressure vessel preheated to 212°C. The vessel was sealed immediately and pressured with water to 3000 ATM. The cooling schedule was as follows:
212° to 191°C 65 minutes
191° to 181°C 63 minutes
181° to 175°C 2 hours
175° to 174°C 6 hours, 26 minutes
174° to 45°C 3 hours, 15 minutes
The rod was cut into test samples and analyzed with the following results:

| DSC (Differential Scanning Calorimetry) | | |
|---|---|---|
| m.p.,°C | Center of Bar | 1 cm from Bar Edge |
| 1st heat | 150.5 | 152.4 |
| 2nd heat | 137.9 | 139.0 |
| ΔT | 12.6 | 13.4 |

| Heat of Fusion | | |
|---|---|---|
| 1st heat | 198.8 J/g | |
| 2nd heat | 134.4 J/g | |

| Infrared Crystallinity Index (Samples cut from within 5 mm of bar edge) | | |
|---|---|---|
| In Bar Direction | 0.613 | |
| Perpendicular to Bar Direction | 0.633 | |

| Flex Modulus (KPSI) | | |
|---|---|---|
| | 424.0 (2923 MPa) | |
| | 386.1 (2662 MPa) | |

| Deformation (Creep) ASTM D621 (% at 1000 psi (6895 kPa) load) | | |
|---|---|---|
| In Bar Direction | 0.4 | |
| Perpendicular to Bar Direction | 0.6 | |

| Density g/ml | | |
|---|---|---|
| Gradient column | 0.9595 | |
| Infra Red | 0.957, 0.958 | |

| Tensile Properties: | In Bar Direction (6" (152 mm) Test Bars) (Type I) | Perpendicular to Bar Direction (2-1/2" (64 mm) Test Bars) (Type V) |
|---|---|---|
| Tensile Strength, PSI (MPa) | | |
| Yield: | 4743 (32.7) | 4516 (31.1) |
| | 4758 (32.8) | 4526 (31.2) |
| Max: | 4743 (32.7) | 5614 (38.7) |
| | 4758 (32.8) | 5005 (34.5) |
| Break: | 4396 (30.3) | 5004 (34.5) |
| | 3695 (25.5) | 5040 (34.8) |

| Tensile Modulus, | | |
|---|---|---|
| KPSI: (MPa) | 611.1 (4214) | 520.3 (3587) |
| | 613.0 (4227) | 513.9 (3543) |

| Elongation, %: | | |
|---|---|---|
| Break | 355 | 433 |
| | 315 | 400 |

| IZOD IMPACT. ft.lb./in. (N/mm) of notch | | |
|---|---|---|
| | Bar Direction | Perpendicular to Bar Direction |
| | 24.8 (4.34) | 26.1 (4.57) |
| | 22.0 (3.85) | 25.0 (4.38) |

### EXAMPLE 10

### Effect of Sequence of Heat-treatment, Cooling, Reheating to a Lower Temperature, and Pressure Recrystallization on UHMWPE.

A UHMW PE bar (3" x 15" - 76 x 381 mm) of the same type as in Example 1 was heated for five hours at 325°C under N₂, then slowly cooled to room temperature. It was reheated to 225°C, and pressure recrystallized as described in Example 9 according to the following schedule:
241° to 191°C 3000 ATM (304 MPa) 2 hours, 15 minutes
191° to 181°C 3000 ATM (304 MPa) 2 hours
181° to 171°C 3000 ATM (304 MPa) 6 hours
The resulting product was machined into test pieces and analyzed with the following results:

| DSC | | |
|---|---|---|
| m.p., °C | Center of Bar | 1 cm in from Bar Edge |
| 1st heat | 149.3 | 149.1 |
| 2nd heat | 134.3 | 135.2 |
| ΔT | 15 | 13.9 |

| Heat of Fusion | | |
|---|---|---|
| 1st heat | 223.6 J/g | 229.6 J/g |
| 2nd heat | 156.1 J/g | 162.3 J/g |

| Infrared Crystallinity Index | | |
|---|---|---|
| In Bar Direction | 0.745 | |
| Perpendicular to Bar Direction | 0.759 | |

| Tensile Properties | | |
|---|---|---|
| Tensile Strength, PSI (MPa) | | |
| At Yield | 4706 (32.4) | 4463 (30.8) |
| At Break | 5362 (37.0) | 5326 (36.7) |

| Tensile Modulus, | | |
|---|---|---|
| KPSI: (MPa) | 649.7 (4480) | 404.2 (2787) |

| Elongation, % | | |
|---|---|---|
| At Yield | 4.7 | 4.5 |
| At Break | 330 | 335 |

| Deformation (Creep) ASTM D621 Test (% at 1000 psi (6895 kPa) load) | | |
|---|---|---|
| | 0.4 | |
| | 0.3 | |

### Effect of Preheating by Reflux

Alternatively, the preliminary heating of Example 10 may be achieved by refluxing in vapors as described below.

A 3" x 18" (76 x 457 mm) rod of UHMWLPE (American Hoechst, Hostalen GUR 415) was heated in refluxing vapors of Krytox®-143AZ (E. I. du Pont de Nemours and Company, Wilmington, Delaware) (at 333-335°C) for 2 hours, 40 minutes. Krytox®-143AZ is a perfluoroalkylpolyether that is a nonflammable, chemically inert liquid having unusually high thermal and oxidative stability. Other materials demonstrating these characteristics may also be suitable. The system was protected by a nitrogen atmosphere and was wrapped with glass insulation to facilitate slow cooling. As compared to the starting material, the resulting product has improved crystallinity (IRCI from 0.27 to 0.47), a tensile modulus (from 210 KPSI to 300 KPSI (1448 to 2069 MPa)), and tensile strength at yield (from 3400 to 3850 psi (23.4 to 26.5 MPa). Most significantly, the product displays a large increase in elongation at break (from 315% to 893%).

When the above described material was recrystallized from 220°C under 3000 ATM (304 MPa), a new polyethylene resulted possessing extremely high elongation at break (667%) along with the high tensile strength at yield (4900 psi (33.8 MPa)) and the tensile modulus (574 KPSI (3958 MPa)) expected of the superior, enhanced UHMWLPE materials.

| | |
|---|---|
| Flex Modulus, KPSI (MPa) | 436.4 (3009) |
| | 431.2 (2973) |
| | 433.80 (av) (2991) |
| Density | .9684 |
| IZOD IMPACT, (ft.lb./in. of notch) | 17.1 (2.99 N/mm) |
| | 15.9 (2.78 N/mm) |
| | 16.5 (av) (2.89 N/mm) |

### EXAMPLE 11

Further evidence shows that UHMWPE polymer pretreated to at least 325°C gives a superior enhanced material (SEUHMW PE).

The purpose of this Example is to show the importance of the preheat temperature.

| | | | | |
|---|---|---|---|---|
| Preheat Temperature(C°C) | 225 | 290 | 310 | 325 |
| Crystallinity Index | .553 | .550 | .605 | 0.76 |
| Heat of Fusion, J/g | 186 | 200 | 190 | 219 |
| Tensile, Yield PSI (MPa) | 4268 (29.4) | 4277 (29.5) | 4212 (29.1) | 4819 (33.2) |
| Deformation (Creep) ASTM D621 Test (% at 1000 psi (6895 kPa) load) | 0.6 | | | 0.4 |
| IZOD ft.lb./in. of Notch (N/mm) | 20.6 (3.6) | 20.7 (3.6) | 20.6 (3.6) | 24 (4.2) |

### Effect of Heating Temperature on UHMW PE

The samples were heated by placing 3/4" (19 mm) cubes of UHMW polyethylene (Hoechst Hostalen GUR 415, m.w. 4-6 million, fabricated by Westlake) wrapped in Teflon® film in a large test tube protected from air with N₂. In the first experiment, a small thermocouple was inserted into the center of the cube, the purpose being to determine the time necessary for the sample to reach test temperature. A plug of glass wool was placed above the sample to control convection currents. The tube was heated with a Wood's metal bath. After the heat treatment was complete, the sample was wrapped in insulation to ensure slow cooling. At a bath temperature of 250°C, the sample required 45 minutes to reach test temperature.

| Time Sample at Temperature hrs:min | Test Temperature °C | Crystallinity Index (by IR) |
|---|---|---|
| 4:00 | 250 | 0.232 |
| 20:00 | 250 | 0.244 |
| 4:00 | 293 | 0.264 |
| :01 | 293 | 0.230 |
| 4:00* | 320-325 | 0.374 |
| 1:00* | 334-335 | 0.378 |
| 1:00* | 340-342 | 0.391 |

| | | |
|---|---|---|
| *Heated by submerging sample wrapped in Teflon® film under Woods metal as described in the following paragraph. | | |

### Effect of Time on Heating UHMW PE

Small cubes (3/4" - 19 mm) of UHMW PE cut from the rod form of Hoechst Hostalen GUR 415 were wrapped in Teflon® film, tied to a glass rod, and pushed under the surface of a Wood's metal bath. In the first experiment, a small thermocouple was inserted in the cube. When plunged into a 322-329°C bath, twelve minutes were required for the sample center to reach 321°C. The time at temperature (not the time in the bath) is recorded below. The samples were removed from the bath and wrapped in glass fiber insulation to permit slow cooling which required 1.5 hours to reach 80°C. The extent of change was determined by measuring crystallinity indices.

| Time Sample (at 320-325°C (hrs:min) | Crystallinity Index by (IR) |
|---|---|
| no heating | .258 |
| 0:10 | .261 |
| 0:20 | .294 |
| 1:00 | .330 |
| 4:00 | .374 |

### Heat Treatment of UHMW PE (Large Scale)

A 3" (76 mm) diameter by 18" (457 mm) bar of UHMW polyethylene (Hoechst Hostalen GUR 415, m.w. 4-6 million, fabrication by Westlake) was heated under nitrogen at 325°C for 4 hours (65B). The bar was cut into test pieces as was a bar of the same starting polymer that had not been treated. Tests were run sequentially.

| DSC | Untreated Polymer | Thermally Treated sharper (narrow curve) | % Difference |
|---|---|---|---|
| m.p.°C | 139.7 | 137.5 | |
| Heat of Fusion J/g | 154.6 | 197.5 | +28 |
| Crystallinity Index (IR) | 0.258 | 0.386 | +50 |

| Tensile Properties | | | |
|---|---|---|---|
| Tensile Strength, psi (MPa) | | | |
| Yield | 3380 (23.3) | 3694 (25.5) | |
| | 3456 (23.8) | 3642 (25.1) | |
| | 3418 (23.6) (av) | 3668 (25.3) (av) | +7.3 |
| Max. | 5361 (37.0) | 4706 (32.4) | |
| | 4864 (33.5) | 4673 (32.2) | |
| | 5113 (35.3) (av) | 4690 (32.3) (av) | |
| Break | 5361 (37.0) | 4705 (32.4) | |
| | 4864 (33.5) | 4673 (32.2) | |
| | 5113 (35.3) (av) | 4689 (av) (32.3) | |
| Elongation, % Break | 330 | 490 | |
| | 300 | 500 | |
| | 315 (av) | 495 (av) | +57 |
| Modulus, KPsi (MPa) | 208.4 (1437) | 244.6 (1687) | |
| | 210.5 (1451) | 253.7 (1749) | |
| | 209.5 (1445) (av) | 249.1 (1718) (av) | +19 |
| Flex Modulus, KPsi (MPa) | 124.4 (858) | 151.5 (1045) | |
| | 137.1 (945) | 146.8 (1012) | |
| | 130.7 (901) (av) | 149.1 (1028) (av) | +14 |
| IZOD IMPACT, (ft.lb./in. of notch) (N/mm) | 15.93 (2.8) | 19.97 (3.5) | |
| | 20.81 (3.6) | 22.68 (4.0) | |
| | 18.37 (av) | 21.32 (av) | +16 |
| | (3.2) | (3.7) | |
| Deformation (Creep) ASTM D621 Test (% at 1000 psi (6895 kPa) load) | 1.8 | 1.6 | -17 |
| | 1.7 | 1.3 | |

Similarly, a 3" diameter bar of different UHMW polyethylene (Himont 1900, m.w. 1,000,000) was heat pretreated in an inert atmosphere, for example, of N₂. The physical properties of the product had greatly inproved elongation and impact resistance.

| DSC | Untreated Polymer | Thermally Treated | % Difference |
|---|---|---|---|
| Heat of Fusion J/g | 166.3 | 190.7 | +15 |
| Crystallinity Index (IR) | .284 | .379 | +33 |

| Tensile Properties | | | |
|---|---|---|---|
| Tensile Strength, psi (MPa) | | | |
| Yield | 3544 (24.4) | 3721 (25.7) | |
| | 3703 (25.5) | 3589 (24.7) | |
| | 3622 (25.0) (av) | 3655 (25.2) (av) | - 0 |
| Max. | 7387 (50.9) | 6545 (45.1) | |
| | 7190 (49.6) | 5999 (41.4) | |
| | 7289 (50.3) (av) | 6272 (43.2) (av) | -14 |
| Elongation, % Break | 200 | 343 | |
| | 216 | 293 | |
| | 208 (av) | 318 (av) | +53 |
| % Yield | 16.6 | 20 | |
| | 20 | 16.6 | |
| Modulus, KPsi (MPa) | 128.4 (885) | 212.7 (1467) | |
| | 216.2 (1491) | 192.7 (1329) | |
| | 202.7 (1398) (av) | 202.7 (1398) (av) | 0 |
| IZOD IMPACT, (ft.lb./in. of notch) (N/mm) | 13.05 (2.3) | 24.26 (4.2) | |
| | 11.94 (2.1) | 17.12 (3.0) | |
| | 12.49 (av) | 21.09 (av) | +65 |
| | (2.2) | (3.7) | |

### EXAMPLE 12

A 3" (76 mm) diameter bar (rod), 18" (457 mm) in length, of American Hoechst Hostalen GUR 415 ultrahigh molecular weight polyethylene, was heated in an oven and then encapsulated with low molecular weight polyethylene by rolling the hot rod onto a 1/16" (1.6 mm) sheet of low molecular weight polyethylene heated to 180°C on a large hot plate. An intervening sheet of "Teflon" Polytetrofluoroethylene film was kept on the encapsulated rod to prevent sticking to the hot plate. The rod ends were similarly sealed. The "Teflon" film was kept on the encapsulated rod to prevent sticking in the reactor.

The bar was heated to 225°C under a nitrogen atmosphere and transfered to the reactor at 225°C. After sealing, the reactor pressure was taken to 3000 atmospheres which caused the temperature to reach 237°C. The reactor was permitted to cool to 180°C in 6.5 h, then maintained at this temperature for 1h. The temperature was dropped to 170°C, held at this temperature for 3h, then cooled slowly to 150°C from where it was cooled rapidly.

The rod, which remained coated, was cut and machined into two test pieces (A and B) which gave the following results:

| DSC | SAMPLE | |
|---|---|---|
| 1st Heat: | A | B |
| Melt point, °C | 149.1 | 153.7 |
| Heat of Fusion, J/g | 219.8 | 209.5 |

| 2nd heat: | | |
|---|---|---|
| Melt point, °C | 135.5 | 136.6 |
| Heat of fusion, J/g | 141.2 | 144.9 |
| Crystallinity Index (IR) | 0.566 | 0.567 |

| Tensile Properties: | | |
|---|---|---|
| Tensile Strength, psi (MPa) | | |
| At Yield | 4149 (28.6) | 4076 (28.1) |
| At Max. | 7448 (51.4) | 8138 (56.1) |
| At Break | 7448 (51.4) | 8138 (56.1) |
| Elongation, % | 323 | 346 |
| Modulus, Kpsi (MPa) | 363.6 (2507) | 358.2 (2470) |
| Creep Deformation, % (D621) | 0.6 | 0.6 |
| IZOD Impact, | 15.9 | 15.8 |
| (ftlb/in. of notch) (N/mm) | (2.8) | (2.8) |

### EXAMPLE 13

A 5.75" segment of enhanced ultrahigh molecular weight polyethylene prepared as in Example 5, was skived to two films (A and B), of 11 mil and 5 mil thickness, respectively. The following properties were obtained (averaged from five tests per film sample):

| Tensile Properties: | SAMPLE | |
|---|---|---|
| Tensile Strength, psi (MPa) | A | B |
| At Yield | 3035 (20.9) | 3108 (21.4) |
| At Max. | 6554 (45.2) | 4083 (28.2) |
| At Break | 6481 (44.7) | 4083 (28.2) |
| At 5% Elongation | 2667 (18.4) | 2705 (18.7) |
| Tensile Modulus, Kpsi (MPa) | 129.7 (894) | 165.6 (1142) |
| Elongation at Break, % | 470 | 237.6 |

The skived films were hot drawn in a tenter frame at 140°C. One piece of the 5 mil (127 µm) film was drawn 6 fold in one direction (C). A second piece of the 5 mil film was drawn 3 fold in both directions (D):

| | SAMPLE | |
|---|---|---|
| Tensile Strength, psi: (MPa) | C | D |
| At Yield | 37,819 (260.1) | 13,720 (94.6) |
| At Max. | 42,058 (290.0) | 19,358 (133.5) |
| At Break | 46,426 (320.1) | 18,995 (131.0) |
| Tensile Modulus, Kpsi (MPa) | 93.3 (643) | 94.9(654) |
| Elongation at Break, % | 56 | 132.4 |
| Thickness, mils (µm) | 2.6 (66) | 1.6 (41) |

### CONTROL B

In this experiment, a product was prepared in the manner of Example 1 of U.S. Patent No. 3,944,536.

A sample of the ultrahigh molecular weight polyethylene used in Example 1 previously described herein, measuring 1/4" x 1/4" x 1/8" (6.4 x 6.4 x 3.2 mm) thick was heated between a film of polytetrafluoroethylene on a hot plate at atmospheric pressure and at a temperature of 160°C on the upper side and 270°C on the lower side.

The molten sample was transferred quickly to a hydraulic press at room temperature where the sample was subjected to a pressure of 8 tons (about 250,000 psi, (1724 MPa) or about 16,000 ATM). It required 1.6 seconds to attain full pressure. The resulting polymer was tested. It displayed an infrared crystallinity index of 0.199. Its melting point was 134.5°C and, when reheated, 134.4°C.

## Claims (Claims for the following Contracting State(s): AT, BE, CH, DE, FR, GB, IT, LU, NL, SE)

1. An ultrahigh molecular weight linear polyethylene having a molecular weight of 400,000 to 10,000,000, a crystalline melting point of greater than 144°C, the reduction in said melting point upon remelting being greater than 11°C, and an infrared crystallinity index of at least about 0.45.

2. The polyethylene of Claim 1 exhibiting a flexural modulus of 250-500 kpsi (1724-3448 MPa), a tensile stress at yield of 3.5-4.5 kpsi (24-31 MPa), a tensile stress at break of 4-9 kpsi (28-62 MPa), a tensile modulus of 300-700 kpsi (2069-4827 MPa), an elongation at break of 200-500%, a notched Izod impact resistance of 12-25 ft. lb. per in. (6.4-13.3 J/cm) of notch and a creep at a compression of 1 kpsi (6.9 MPa) of less than 1% after 24 hours at a temperature of 23°C and a relative humidity of 50%.

3. The polyethylene of Claim 2 wherein the tensile modulus is 300-600 kpsi (2069-4137 MPa) and the infrared crystallinity index is at least 0.5.

4. A process for preparing the polyethylene of Claim 2 consisting essentially of the following steps:
(a) forming an article of an ultrahigh molecular weight linear polyethylene having a molecular weight of 400,000 to 10,000,000;
(b) subjecting said article to a fluid under pressure of at least 284 MPa and a temperature of at least 190°C for at least 0.5 hour;
(c) reducing the temperature to about 160-170°C or lower while maintaining the pressure at at least 284 MPa, the rate of reduction in temperature being such that temperature gradients in the shaped article are substantially avoided; and
(d) cooling to a temperature of about 130°C or lower and reducing the pressure to about 101 kPa in a manner such that remelting of said article is prevented.

5. The polyethylene of Claim 1 wherein the flexural modulus is 250-650 kpsi (1724-4482 MPa), the tensile stress at yield is 3.5-4.5 kpsi (24-37 MPa), the tensile stress at break is 4-6 kpsi (27.6-41.4 MPa), the tensile modulus is 300-700 kpsi (2069-4827 MPa), the elongation at break is 200-600% the notched IZOD impact resistance is 12-25 ft. lb. per inch (6.4-13.3 J/cm) of notch and creep at a compression of 1 kpsi (6.9 MPa) is less than 1% after 24 hours at a temperature of 23°C and a relative humidity of 50%.

6. The polyethylene of Claim 5 wherein the tensile modulus is 300-650 kpsi (2069-4482 MPa) and the infrared crystallinity is at least 0.5.

7. A process for preparing the polyethylene of Claim 5 consisting essentially of the following steps:
(a) forming an article of an ultrahigh molecular weight linear polyethylene having a molecular weight of 400,000 to 10,000,000;
(b) subjecting said article to a preliminary heat treatment at a temperature of 320-340°C, in an inert atmosphere for at least 0.5 hour;
(c) subjecting said article to a fluid under pressure of at least 2800 atm (284 MPa) and a temperature of at least 190°C for at least 0.5 hour;
(d) reducing the temperature to 160°-170°C or lower while maintaining the pressure at at least 284 MPa, the rate of reduction in temperature being such that temperature gradients in the shaped article are substantially avoided; and
(e) cooling to a temperature of about 130°C or lower and reducing the pressure to approximately 101 kPa in a manner such that remelting of said article is prevented.

8. The process of Claim 4 or 7 wherein step (a) is performed after step (d) or (e), respectively.

9. The process of Claim 4, 7 or 8 wherein said fluid is water.

10. The process of Claim 7 wherein said heat treatment in step (b) is continued for at least 3 hours at a temperature of at least 320°C.

11. The process of Claim 4 or 7 wherein said pressure in step (b) or (c), respectively, is at least 304 MPa

12. The process of Claim 4 or 7 wherein said temperature in step (b) or (c), respectively, is 200°-230°C.

13. The process of Claim 4 or 7 wherein the temperature and pressure in step (d) or (e), respectively, are maintained for at least one hour.

14. The process of Claim 4 or 7 wherein the surface of the article is shaved after step (d) or (e), respectively.

15. The process of Claim 4 or 7 wherein the cooling rate in step (c) or (d), respectively, is no greater than 35°C per hour.

16. The process of Claim 15 wherein the cooling rate in step (d) is no greater than 10°C per hour.

17. An improved folded chain ultrahigh molecular weight - linear polyethylene having a molecular weight of 400,000 to 10,000,000, and exhibiting a flexural modulus of 150-300 kpsi (1034-2069 MPa), a tensile stress at yield of 3.5-4.0 kpsi (24-28 MPa), a tensile stress at break of 4-6 kpsi (28-41 MPa), a tensile modulus of 150-300 kpsi, a notched Izod impact resistance of 8.0-13.3 J/cm 15-25 ft. lb. per in. of notch, an elongation at break of 200-800%, a creep at a compression of 1 kpsi (6.9 MPa) of less than 2% after 24 hours at a temperature of 23°C and a relative humidity of 50%, and an infrared crystallinity index of at least about 0.35.

18. The polyethylene of Claim 17 having a molecular weight of at least 1,000,000.

19. An article consisting essentially of the polyethylene of any one of Claims 1 to 3, 5, 6, 17, or 18.

20. The article of Claim 19 wherein its dimensions are at least one inch by at least one inch (2.54 by 2.54 cm).

21. The article of Claim 19 wherein its smallest dimension is at least 0.2 inch (5 mm).

22. A process for preparing the polyethylene of Claim 17 consisting essentially of the following steps:
(a) forming an article of an ultrahigh molecular weight linear polyethylene having a molecular weight of 400,000;
(b) subjecting said article to a temperature of 190-340°C in an inert atmosphere for at least 0.5 hour;
(c) cooling the article non-precipitously to a temperature of about 130°C or below.

23. The process of Claim 22 wherein step (a) is performed after step (c).

24. The process of Claim 22 or 23 wherein said temperature in step (b) is 320°-340°C.

25. The process of Claim 22, 23 or 24 wherein the temperature in step (b) is maintained for at least one hour.

## Claims (Claims for the following Contracting State(s): ES)

1. A process for preparing an ultrahigh molecular weight linear polyethylene having a molecular weight of 400,000 to 10,000,000, a crystalline melting point of greater than 144°C, the reduction in said melting point upon remelting being greater than 11°C, and an infrared crystallinity index of at least about 0.45, consisting essentially of the following steps:
(a) forming an article of an ultrahigh molecular weight linear polyethylene having a molecular weight of 400,000 to 10,000,000;
(b) subjecting said article to a fluid under pressure of at least 284 MPa and a temperature of at least 190°C for at least 0.5 hour;
(c) reducing the temperature to about 160-170°C or lower while maintaining the pressure at at least 284 MPa, the rate of reduction in temperature being such that temperature gradients in the shaped article are substantially avoided; and
(d) cooling to a temperature of about 130°C or lower and reducing the pressure to about 101 kPa in a manner such that remelting of said article is prevented.

2. A process for preparing a polyethylene wherein the flexural modulus is 250-650 kpsi (1724-4482 MPa), the tensile stress at yield is 3.5-5.4 kpsi (24-37 MPa), the tensile stress at break is 4-6 kpsi (27.6-41.4 MPa), the tensile modulus is 300-700 kpsi (2069-4827 MPa), the elongation at break is 200-600%, the notched IZOD impact resistance is 12-25 ft. lb. per inch (6.4-13.3 J/cm) of notch and creep at a compression of 1 kpsi (6.9 MPa) is less than 1% after 24 hours at a temperature of 23°C and a relative humidity of 50% consists essentially of the following steps:
(a) forming an article of an ultrahigh molecular weight linear polyethylene having a molecular weight of 400,000 to 10,000,000;
(b) subjecting said article to a preliminary heat treatment at a temperature of 320-340°C, in an inert atmosphere for at least 0.5 hours;
(c) subjecting said article to a fluid under pressure of at least 2800 atm. (284 MPa) and a temperature of at least 190°C for at least 0.5 hours;
(d) reducing the temperature to 160-170°C or lower while maintaining the pressure at at least 284 MPa, the rate of reduction in temperature being such that temperature gradients in the shaped article are substantially avoided; and
(e) cooling to a temperature of about 130°C or lower and reducing the pressure to approximately 101 kPa in a manner such that remelting of said article is prevented.

3. The process of either of claims 1 and 2 wherein step (a) is performed after step (d) or (e), respectively.

4. The process of any one of claims 1, 2 or 3 wherein said fluid is water.

5. The process of claim 2 wherein said heat treatment in step (b) is continued for at least 3 hours at a temperature of at least 320°C.

6. The process of either of claims 1 and 2 wherein said pressure in step (b) or (c), respectively, is at least 304 MPa.

7. The process of either of claims 1 and 2 wherein said temperature in step (b) or (c), respectively, is 200-230°C.

8. The process of either of claims 1 and 2 wherein the temperature and pressure in step (d) or (e), respectively, are maintained for at least one hour.

9. The process of either of claims 1 and 2 wherein the surface of the article is shaved after step (d) or (e), respectively.

10. The process of either of claims 1 and 2 wherein the cooling rate in step (c) or (d), respectively, is no greater than 35°C per hour.

11. The process of claim 10 wherein the cooling rate in step (d) is no greater than 10°C per hour.

12. A process for preparing an improved folded chain ultrahigh molecular weight linear polyethylene having a molecular weight of 400,000 to 10,000 000, and exhibiting a flexural modulus of 150-300 kpsi (1034-2069 MPa), a tensile stress at yield of 3.5-4.0 kpsi (24-28 MPa), a tensile stress at break of 4-6 kpsi (28-41 MPa), a tensile modulus of 150-300 kpsi, a notched Izod impact resistance of 15-25 ft. lb. per in. (8-13.3 J/cm) of notch, an elongation at break of 200-800%, a creep at a compression of 1 kpsi (6.9 MPa) of less than 2% after 24 hours at a temperature of 23°C and a relative humidity of 50%, and an infrared crystallinity index of at least about 0.35, consisting essentially of the following steps:
(a) forming an article of an ultrahigh molecular weight linear polyethylene having a molecular weight of 400,000;
(b) subjecting said article to a temperature of 190-340°C in an inert atmosphere for at least 0.5 hour;
(c) cooling the article non-precipitously to a temperatureof about 130°C or below.

13. The process of claim 12 wherein step (a) is performed after step (c).

14. The process of either of claims 12 and 13 wherein said temperature in step (b) is 320°-340°C.

15. The process of any one of claims 12, 13 and 14, wherein the temperature in step (b) is maintained for at least one hour.

## Patentansprüche (Patentansprüche für folgende(n) Vertragsstaat(en): AT, BE, CH, DE, FR, GB, IT, LU, NL, SE)

1. Lineares ultrahochmolekulares Polyethylen mit einem Molekulargewicht von 400 000 bis 10 000 000, einem kristallinen Schmelzpunkt von mehr als 144°C, wobei die Erniedrigung des Schmelzpunkts beim Wiederaufschmelzen größer als 11°C ist, und einem Infrarot-Kristallinitätsindex von wenigstens etwa 0,45.

2. Polyethylen gemäß Anspruch 1, das einen Biegemodul von 250-500 kpsi (1724-3448 MPa), eine Streckspannung von 3,5-4,5 kpsi (24-31 MPa), eine Bruchspannung von 4-9 kpsi (28-62 MPa), einen Zugmodul von 300-700 kpsi (2069-4827 MPa), eine Bruchdehnung von 200-500%, eine Izod-Kerbschlagzähigkeit von 12-25 ft.lb. pro inch (6,4-13,3 J/cm) der Kerbe und ein Kriechen bei einer Kompression von 1 kpsi (6,9 MPa) von weniger als 1% nach 24 Stunden bei einer Temperatur von 23°C und einer relativen Feuchtigkeit von 50% hat.

3. Polyethylen gemäß Anspruch 2, worin der Zugmodul 300-600 kpsi (2069-4137 MPa) und der Infrarot-Kristallinitätsindex wenigstens 0,5 ist.

4. Verfahren zur Herstellung des Polyethylens gemäß Anspruch 2, das im wesentlichen die folgenden Stufen umfaßt:
(a) Bildung eines Gegenstandes aus einem linearen ultrahochmolekularen Polyethylen mit einem Molekulargewicht von 400 000 bis 10 000 000,
(b) Einwirkenlassen eines Fluids unter einem Druck von wenigstens 284 MPa und bei einer Temperatur von wenigstens 190°C während wenigstens 0,5 Stunden auf den Gegenstand,
(c) Vermindern der Temperatur auf etwa 160-170°C oder weniger, während der Druck bei wenigstens 284 MPa gehalten wird, wobei die Geschwindigkeit der Temperaturverminderung derartig ist, daß Temperaturgradienten in dem geformten Gegenstand im wesentlichen vermieden werden, und
(d) Abkühlen auf eine Temperatur von etwa 130°C oder weniger und Reduzierung des Drucks auf etwa 101 kPa auf eine derartige Weise, daß Wiederaufschmelzen des Gegenstandes vermieden wird.

5. Polyethylen gemäß Anspruch 1, worin der Biegemodul 250-650 kpsi (1724-4482 MPa), die Streckspannung 3,5-5,4 kpsi (24-37 MPa), die Bruchspannung 4-6 kpsi (27,6-41,4 MPa), der Zugmodul 300-700 kpsi (2069-4827 MPa), die Bruchdehnung 200-600%, die Izod-Kerbschlagzähigkeit 12-25 ft. lb. pro inch (6,4-13,3 J/cm) der Kerbe und das Kriechen bei einer Kompression von 1 kpsi (6,9 MPa) weniger als 1% nach 24 Stunden bei einer Temperatur von 23°C und einer relativen Feuchtigkeit von 50% ist.

6. Polyethylen gemäß Anspruch 5, worin der Zugmodul 300-650 kpsi (2069-4482 MPa) und der Infrarot-Kristallinitätsindex wenigstens 0,5 ist.

7. Verfahren zur Herstellung des Polyethylens gemäß Anspruch 5, das im wesentlichen die folgenden Stufen umfaßt:
(a) Bildung eines Gegenstandes aus einem linearen ultrahochmolekularen Polyethylen mit einem Molekulargewicht von 400 000 bis 10 000 000,
(b) Einwirkenlassen einer vorhergehenden Wärmebehandlung bei einer Temperatur von 320-340°C in einer inerten Atmosphäre während wenigstens 0,5 Stunden auf den Gegenstand,
(c) Einwirkenlassen eines Fluids unter einem Druck von wenigstens 2800 atm (284 MPa) und bei einer Temperatur von wenigstens 190°C während wenigstens 0,5 Stunden auf den Gegenstand,
(d) Vermindern der Temperatur auf etwa 160-170°C oder weniger, während der Druck bei wenigstens 284 MPa gehalten wird, wobei die Geschwindigkeit der Temperaturverminderung derartig ist, daß Temperaturgradienten in dem geformten Gegenstand im wesentlichen vermieden werden, und
(e) Abkühlen auf eine Temperatur von etwa 130°C oder weniger und Reduzierung des Drucks auf etwa 101 kPa auf eine derartige Weise, daß Wiederaufschmelzen des Gegenstandes vermieden wird.

8. Verfahren gemäß Anspruch 4 oder 7, worin die Stufe (a) nach der Stufe (d) bzw. Stufe (e) durchgeführt wird.

9. Verfahren gemäß Anspruch 4, 7 oder 8, worin das Fluid Wasser ist.

10. Verfahren gemäß Anspruch 7, worin die Wärmebehandlung in Stufe (b) wenigstens 3 Stunden bei einer Temperatur von wenigstens 320°C weitergeführt wird.

11. Verfahren gemäß Anspruch 4 oder 7, worin der Druck in Stufe (b) bzw. Stufe (c) wenigstens 304 MPa ist.

12. Verfahren gemäß Anspruch 4 oder 7, worin die Temperatur in Stufe (b) bzw. Stufe (c) 200-230°C ist.

13. Verfahren gemäß Anspruch 4 oder 7, worin die Temperatur und der Druck in Stufe (d) bzw. Stufe (e) wenigstens eine Stunde beibehalten werden.

14. Verfahren gemäß Anspruch 4 oder 7, worin die Oberfläche des Gegenstandes nach Stufe (d) bzw. Stufe (e) abgeschabt wird.

15. Verfahren gemäß Anspruch 4 oder 7, worin die Abkühlungsgeschwindigkeit in der Stufe (c) bzw. der Stufe (d) nicht größer als 35°C pro Stunde ist.

16. Verfahren gemäß Anspruch 15, worin die Abkühlungsgeschwindigkeit in der Stufe (d) nicht größer als 10°C pro Stunde ist.

17. Verbessertes lineares ultrahochmolekulares Polyethylen mit gefalteter Kette, das ein Molekulargewicht von 400 000 bis 10 000 000 hat, und das einen Biegemodul von 150-300 kpsi (1034-2069 MPa), eine Streckspannung von 3,5-4,0 kpsi (24-28 MPa), eine Bruchspannung von 4-6 kpsi (28-41 MPa), einen Zugmodul von 150-300 kpsi, eine Izod-Kerbschlagzähigkeit von 15-25 ft.lb. pro inch (8 -13,3 J/cm) der Kerbe, eine Bruchdehnung von 200-800%, ein Kriechen bei einer Kompression von 1 kpsi (6,9 MPa) von weniger als 2% nach 24 Stunden bei einer Temperatur von 23°C und einer relativen Feuchtigkeit von 50%, und einen Infrarot-Kristallinitätsindex von wenigstens 0,35 hat.

18. Polyethylen gemäß Anspruch 17, das ein Molekulargewicht von wenigstens 1 000 000 hat.

19. Gegenstand, der im wesentlichen aus dem Polyethylen gemäß irgendeinem der Ansprüche 1 bis 3, 5, 6, 17 oder 18 besteht.

20. Gegenstand gemäß Anspruch 19, worin seine Dimensionen wenigstens ein inch mal wenigstens ein inch (2,54 cm mal 2,54 cm) sind.

21. Gegenstand gemäß Anspruch 19, worin seine kleinste Dimension wenigstens 0,2 inch (5 mm) ist.

22. Verfahren zur Herstellung des Polyethylens gemäß Anspruch 17, das im wesentlichen die folgenden Stufen umfaßt:
(a) Bilden eines Gegenstandes aus einem linearen ultrahochmolekularen Polyethylen mit einem Molekulargewicht von 400 000,
(b) Einwirkenlassen einer Temperatur von 190-340°C in einer inerten Atmosphäre während wenigstens 0,5 Stunden auf den Gegenstand,
(c) Nicht stark-abfallendes Abkühlen des Gegenstandes auf eine Temperatur von etwa 130°C oder weniger.

23. Verfahren gemäß Anspruch 22, worin die Stufe (a) nach der Stufe (c) durchgeführt wird.

24. Verfahren gemäß Anspruch 22 oder 23, worin die Temperatur in Stufe (b) 320-340°C ist.

25. Verfahren gemäß Anspruch 22, 23 oder 24, worin die Temperatur in Stufe (b) wenigstens eine Stunde beibehalten wird.

## Patentansprüche (Patentansprüche für folgende(n) Vertragsstaat(en): ES)

1. Verfahren zur Herstellung eines linearen ultrahochmolekularen Polyethylens mit einem Molekulargewicht von 400 000 bis 10 000 000, einem kristallinen Schmelzpunkt von mehr als 144°C, wobei die Erniedrigung des Schmelzpunkts beim Wiederaufschmelzen größer als 11°C ist, und einem Infrarot-Kristallinitätsindex von wenigstens etwa 0,45, das im wesentlichen die folgenden Stufen umfaßt:
(a) Bildung eines Gegenstandes aus einem linearen ultrahochmolekularen Polyethylen mit einem Molekulargewicht von 400 000 bis 10 000 000,
(b) Einwirkenlassen eines Fluids unter einem Druck von wenigstens 284 MPa und bei einer Temperatur von wenigstens 190°C während wenigstens 0,5 Stunden auf den Gegenstand,
(c) Vermindern der Temperatur auf etwa 160-170°C oder weniger, während der Druck bei wenigstens 284 MPa gehalten wird, wobei die Geschwindigkeit der Temperaturverminderung derartig ist, daß Temperaturgradienten in dem geformten Gegenstand im wesentlichen vermieden werden, und
(d) Abkühlen auf eine Temperatur von etwa 130°C oder weniger und Reduzierung des Drucks auf etwa 101 kPa auf eine derartige Weise, daß Wiederaufschmelzen des Gegenstandes vermieden wird.

2. Verfahren zur Herstellung eines Polyethylens, worin der Biegemodul 250-650 kpsi (1724-4482 MPa), die Streckspannung 3,5-5,4 kpsi (24-37 MPa), die Bruchspannung 4-6 kpsi (27,6-41,4 MPa), der Zugmodul 300-700 kpsi (2069-4827 MPa), die Bruchdehnung 200-600%, die Izod-Kerbschlagzähigkeit 12-25 ft.lb. pro inch (6,4-13,3 J/cm) der Kerbe und das Kriechen bei einer Kompression von 1 kpsi (6,9 MPa) weniger als 1% nach 24 Stunden bei einer Temperatur von 23°C und einer relativen Feuchtigkeit von 50% ist, das im wesentlichen die folgenden Stufen umfaßt:
(a) Bildung eines Gegenstandes aus einem linearen ultrahochmolekularen Polyethylen mit einem Molekulargewicht von 400 000 bis 10 000 000,
(b) Einwirkenlassen einer vorhergehenden Wärmebehandlung bei einer Temperatur von 320-340°C in einer inerten Atmosphäre während wenigstens 0,5 Stunden auf den Gegenstand,
(c) Aussetzen des Gegenstandes an ein Fluid unter einem Druck von wenigstens 2800 atm (284 MPa) und bei einer Temperatur von wenigstens 190°C während wenigstens 0,5 Stunden,
(d) Vermindern der Temperatur auf etwa 160-170°C oder weniger, während der Druck bei wenigstens 284 MPa gehalten wird, wobei die Geschwindigkeit der Temperaturverminderung derartig ist, daß Temperaturgradienten in dem geformten Gegenstand im wesentlichen vermieden werden, und
(e) Abkühlen auf eine Temperatur von etwa 130°C oder weniger und Reduzierung des Drucks auf etwa 101 kPa auf eine derartige Weise, daß Wiederaufschmelzen des Gegenstandes vermieden wird.

3. Verfahren gemäß sowohl Anspruch 1 als auch Anspruch 2, worin die Stufe (a) nach der Stufe (d) bzw. Stufe (e) durchgeführt wird.

4. Verfahren gemäß irgendeinem der Ansprüche 1, 2 oder 3, worin das Fluid Wasser ist.

5. Verfahren gemäß Anspruch 2, worin die Wärmebehandlung in Stufe (b) wenigstens 3 Stunden bei einer Temperatur von wenigstens 320°C weitergeführt wird.

6. Verfahren gemäß sowohl Anspruch 1 als auch Anspruch 2 , worin der Druck in Stufe (b) bzw. Stufe (c) wenigstens 304 MPa ist.

7. Verfahren gemäß sowohl Anspruch 1 als auch Anspruch 2, worin die Temperatur in Stufe (b) bzw. Stufe (c) 200-230°C ist.

8. Verfahren gemäß sowohl Anspruch 1 als auch Anspruch 2, worin die Temperatur und der Druck in Stufe (d) bzw. Stufe (e) wenigstens eine Stunde beibehalten werden.

9. Verfahren gemäß sowohl Anspruch 1 als auch Anspruch 2, worin die Oberfläche des Gegenstandes nach Stufe (d) bzw. Stufe (e) abgeschabt wird.

10. Verfahren gemäß sowohl Anspruch 1 als auch Anspruch 2, worin die Abkühlungsgeschwindigkeit in der Stufe (c) bzw. der Stufe (d) nicht größer als 35°C pro Stunde ist.

11. Verfahren gemäß Anspruch 10, worin die Abkühlungsgeschwindigkeit in der Stufe (d) nicht größer als 10°C pro Stunde ist.

12. Verfahren zur Herstellung eines verbesserten linearen ultrahochmolekularen Polyethylens mit gefalteter Kette, das ein Molekulargewicht von 400 000 bis 10 000 000 hat, und das einen Biegemodul von 150-300 kpsi (1034-2069 MPa), eine Streckspannung von 3,5-4,0 kpsi (24-28 MPa), eine Bruchspannung von 4-6 kpsi (28-41 MPa), einen Zugmodul von 150-300 kpsi, eine Izod-Kerbschlagzähigkeit von 15-25 ft.lb. pro inch (8-13,3 J/cm) der Kerbe, eine Bruchdehnung von 200-800%, ein Kriechen bei einer Kompression von 1 kpsi (6,9 MPa) von weniger als 2% nach 24 Stunden bei einer Temperatur von 23°C und einer relativen Feuchtigkeit von 50%, und einen Infrarot-Kristallinitätsindex von wenigstens 0,35 hat, das im wesentlichen die folgenden Stufen umfaßt:
(a) Bilden eines Gegenstandes aus einem linearen ultrahochmolekularen Polyethylen mit einem Molekulargewicht von 400 000,
(b) Einwirkenlassen einer Temperatur von 190-340°C in einer inerten Atmosphäre während wenigstens 0,5 Stunden auf den Gegenstand,
(c) Nicht stark-abfallendes Abkühlen des Gegenstandes auf eine Temperatur von etwa 130°C oder weniger.

13. Verfahren gemäß Anspruch 12, worin die Stufe (a) nach der Stufe (c) durchgeführt wird.

14. Verfahren gemäß sowohl Anspruch 12 als auch Anspruch 13, worin die Temperatur in Stufe (b) 320-340°C ist.

15. Verfahren gemäß irgendeinem der Ansprüche 12, 13 oder 14, worin die Temperatur in Stufe (b) wenigstens eine Stunde beibehalten wird.

## Revendications (Revendications pour l'(les) Etat(s) contractant(s) suivant(s): AT, BE, CH, DE, FR, GB, IT, LU, NL, SE)

1. Un polyéthylène linéaire de poids moléculaire ultra-élevé, ayant un poids moléculaire de 400 000 à 10 000 000, un point de fusion cristalline supérieur à 144°C, l'abaissement dudit point de fusion par refonte étant supérieur à 11°C, et un indice de cristallinité en infrarouge d'au moins environ 0,45.

2. Le polyéthylène de la revendication 1 présentant un module en flexion de 1724 à 3448 MPa, une contrainte de traction à la limite élastique de 24 à 31 MPa, une contrainte de traction à la rupture de 28 à 62 MPa, un module en traction de 2069 à 4827 MPa, un allongement à la rupture de 200 à 500 %, une résistance au choc Izod avec entaille de 6,4 à 13,3 J/cm d'entaille et un fluage à une compression de 6,9 MPa inférieur à 1 % après 24 heures à une température de 23°C et une humidité relative de 50 %.

3. Le polyéthylène de la revendication 2, dans lequel le module en traction est de 2069 à 4137 MPa et l'indice de cristallinité en infrarouge est d'au moins 0,5.

4. Un procédé pour préparer le polyéthylène de la revendication 2, consistant essentiellement en les étapes suivantes :
(a) former un article d'un polyéthylène linéaire de poids moléculaire ultra-élevé ayant un poids moléculaire de 400 000 à 10 000 000 ;
(b) soumettre ledit article à un fluide sous une pression d'au moins 284 MPa et à une température d'au moins 190°C pendant au moins 0,5 heure ;
(c) abaisser la température à environ 160-170°C ou moins tout en maintenant la pression à au moins 284 MPa, la vitesse d'abaissement de la température étant telle que des gradients de température dans l'article façonné soient sensiblement évités ; et
(d) refroidir à une température d'environ 130°C ou moins et réduire la pression à environ 101 kPa de manière à éviter une refonte dudit article.

5. Le polyéthylène de la revendication 1, dans lequel le module en flexion est de 1724 à 4482 MPa, la contrainte de traction à la limite élastique est de 24 à 37 MPa, la contrainte de traction à la rupture est de 27,6 à 41,4 MPa, le module en traction est de 2069 à 4827 MPa, l'allongement à la rupture est de 200 à 600 %, la résistance au choc Izod avec entaille est de 6,4 à 13,3 J/cm d'entaille et le fluage à une compression de 6,9 MPa est inférieur à 1 % après 24 heures à une température de 23°C et une humidité relative de 50 %.

6. Le polyéthylène de la revendication 5, dans lequel le module en traction est de 2069 à 4482 MPa et la cristallinité en infrarouge est d'au moins 0,5.

7. Un procédé pour préparer le polyéthylène de la revendication 5, consistant essentiellement en les étapes suivantes :
(a) former un article d'un polyéthylène linéaire de poids moléculaire ultra-élevé ayant un poids moléculaire de 400 000 à 10 000 000 ;
(b) soumettre ledit article à un traitement thermique préliminaire à une température de 320 à 340°C dans une atmosphère inerte pendant au moins 0,5 heure ;
(c) soumettre ledit article à un fluide sous une pression d'au moins 284 MPa et à une température d'au moins 190°C pendant au moins 0,5 heure ;
(d) abaisser la température à 160°-170°C ou moins tout en maintenant la pression à au moins 284 MPa, la vitesse d'abaissement de la température étant telle que des gradients de température dans l'article façonné soient sensiblement évités ; et
(e) refroidir à une température d'environ 130°C ou moins et réduire la pression à environ 101 kPa de manière à éviter une refonte dudit article.

8. Le procédé de la revendication 4 ou 7, dans lequel l'étape (a) est exécutée après l'étape (d) ou (e), respectivement.

9. Le procédé de la revendication 4, 7 ou 8, dans lequel ledit fluide est l'eau.

10. Le procédé de la revendication 7, dans lequel ledit traitement thermique de l'étape (b) est poursuivi pendant au moins 3 heures à une température d'au moins 320°C.

11. Le procédé de la revendication 4 ou 7, dans lequel ladite pression dans l'étape (b) ou (c), respectivement, est d'au moins 304 MPa.

12. Le procédé de la revendication 4 ou 7, dans lequel ladite température dans l'étape (b) ou (c), respectivement, est de 200° à 230°C.

13. Le procédé de la revendication 4 ou 7, dans lequel la température et la pression dans l'étape (d) ou (e), respectivement, sont maintenues pendant au moins 1 heure.

14. Le procédé de la revendication 4 ou 7, dans lequel la surface de l'article est rognée après l'étape (d) ou (e), respectivement.

15. Le procédé de la revendication 4 ou 7, dans lequel la vitesse de refroidissement dans l'étape (c) ou (d), respectivement, n'est pas supérieure à 35°C par heure.

16. Le procédé de la revendication 15, dans lequel la vitesse de refroidissement dans l'étape (d) n'est pas supérieure à 10°C par heure.

17. Un polyéthylène linéaire de poids moléculaire ultra-élevé à chaîne plissée amélioré ayant un poids moléculaire de 400 000 à 10 000 000 et présentant un module en flexion de 1034 à 2069 MPa, une contrainte de traction à la limite élastique de 24 à 28 MPa, une contrainte de traction à la rupture de 28 à 41 MPa, un module en traction de 1034 à 2069 MPa, une résistance au choc Izod avec entaille de 8,0 à 13,3 J/cm d'entaille, un allongement à la rupture de 200 à 800 %, un fluage à une compression de 6,9 MPa inférieur à 2 % après 24 heures à une température de 23°C et une humidité relative de 50 %, et un indice de cristallinité en infrarouge d'au moins environ 0,35.

18. Le polyéthylène de la revendication 17, ayant un poids moléculaire d'au moins 1 000 000.

19. Un article constitué essentiellement du polyéthylène de l'une quelconque des revendications 1 à 3, 5, 6, 17 ou 18.

20. L'article de la revendication 19, dont les dimensions sont d'au moins 2,54 sur 2,54 cm.

21. L'article de la revendication 19, dont la plus petite dimension est d'au moins 5 mm.

22. Un procédé pour préparer le polyéthylène de la revendication 17, consistant essentiellement en les étapes suivantes :
(a) former un article d'un polyéthylène linéaire de poids moléculaire ultra-élevé ayant un poids moléculaire de 400 000 ;
(b) soumettre ledit article à une température de 190 à 340°C dans une atmosphère inerte pendant au moins 0,5 heure ;
(c) refroidir l'article sans hâte jusqu'à une température d'environ 130°C ou moins.

23. Le procédé de la revendication 22, dans lequel l'étape (a) est exécutée après l'étape (c).

24. Le procédé de la revendication 22 ou 23, dans lequel ladite température dans l'étape (b) est de 320° à 340°C.

25. Le procédé de la revendication 22, 23 ou 24, dans lequel la température dans l'étape (b) est maintenue pendant au moins 1 heure.

## Revendications (Revendications pour l'(les) Etat(s) contractant(s) suivant(s): ES)

1. Un procédé de préparation d'un polyéthylène linéaire de poids moléculaire ultra-élevé, ayant un poids moléculaire de 400 000 à 10 000 000, un point de fusion cristalline supérieur à 144°C, l'abaissement dudit point de fusion par refonte étant supérieur à 11°C, et un indice de cristallinité en infrarouge d'au moins environ 0,45, consistant essentiellement en les étapes suivantes :
(a) former un article d'un polyéthylène linéaire de poids moléculaire ultra-élevé ayant un poids moléculaire de 400 000 à 10 000 000 ;
(b) soumettre ledit article à un fluide sous une pression d'au moins 284 MPa et à une température d'au moins 190°C pendant au moins 0,5 heure ;
(c) abaisser la température à environ 160-170°C ou moins tout en maintenant la pression à au moins 284 MPa, la vitesse d'abaissement de la température étant telle que des gradients de température dans l'article façonné soient sensiblement évités ; et
(d) refroidir à une température d'environ 130°C ou moins et réduire la pression à environ 101 kPa de manière à éviter une refonte dudit article.

2. Un procédé de préparation d'un polyéthylène dont le module en flexion est de 1724 à 4482 MPa, la contrainte de traction à la limite élastique est de 24 à 37 MPa, la contrainte de traction à la rupture est de 27,6 à 41,4 MPa, le module en traction est de 2069 à 4827 MPa, l'allongement à la rupture est de 200 à 600 %, la résistance au choc Izod avec entaille est de 6,4 à 13,3 J/cm d'entaille et le fluage à une compression de 6,9 MPa est inférieur à 1 % après 24 heures à une température de 23°C et une humidité relative de 50 %, consistant essentiellement en les étapes suivantes :
(a) former un article d'un polyéthylène linéaire de poids moléculaire ultra-élevé ayant un poids moléculaire de 400 000 à 10 000 000 ;
(b) soumettre ledit article à un traitement thermique préliminaire à une température de 320 à 340°C dans une atmosphère inerte pendant au moins 0,5 heure ;
(c) soumettre ledit article à un fluide sous une pression d'au moins 284 MPa et à une température d'au moins 190°C pendant au moins 0,5 heure ;
(d) abaisser la température à 160°-170°C ou moins tout en maintenant la pression à au moins 284 MPa, la vitesse d'abaissement de la température étant telle que des gradients de température dans l'article façonné soient sensiblement évités ; et
(e) refroidir à une température d'environ 130°C ou moins et réduire la pression à environ 101 kPa de manière à éviter une refonte dudit article.

3. Le procédé de la revendication 1 ou 2, dans lequel l'étape (a) est exécutée après l'étape (d) ou (e), respectivement.

4. Le procédé de la revendication 1, 2 ou 3, dans lequel ledit fluide est l'eau.

5. Le procédé de la revendication 2, dans lequel ledit traitement thermique de l'étape (b) est poursuivi pendant au moins 3 heures à une température d'au moins 320°C.

6. Le procédé de la revendication 1 ou 2, dans lequel ladite pression dans l'étape (b) ou (c), respectivement, est d'au moins 304 MPa.

7. Le procédé de la revendication 1 ou 2, dans lequel ladite température dans l'étape (b) ou (c), respectivement, est de 200° à 230°C.

8. Le procédé de la revendication 1 ou 2, dans lequel la température et la pression dans l'étape (d) ou (e), respectivement, sont maintenues pendant au moins 1 heure.

9. Le procédé de la revendication 1 ou 2, dans lequel la surface de l'article est rognée après l'étape (d) ou (e), respectivement.

10. Le procédé de la revendication 1 ou 2, dans lequel la vitesse de refroidissement dans l'étape (c) ou (d), respectivement, n'est pas supérieure à 35°C par heure.

11. Le procédé de la revendication 10, dans lequel la vitesse de refroidissement dans l'étape (d) n'est pas supérieure à 10°C par heure.

12. Un procédé de préparation d'un polyéthylène linéaire de poids moléculaire ultra-élevé à chaîne plissée amélioré ayant un poids moléculaire de 400 000 à 10 000 000 et présentant un module en flexion de 1034 à 2069 MPa, une contrainte de traction à la limite élastique de 24 à 28 MPa, une contrainte de traction à la rupture de 28 à 41 MPa, un module en traction de 1034 à 2069 MPa, une résistance au choc Izod avec entaille de 8,0 à 13,3 J/cm d'entaille, un allongement à la rupture de 200 à 800 %, un fluage à une compression de 6,9 MPa inférieur à 2 % après 24 heures à une température de 23°C et une humidité relative de 50 %, et un indice de cristallinité en infrarouge d'au moins environ 0,35, consistant essentiellement en les étapes suivantes :
(a) former un article d'un polyéthylène linéaire de poids moléculaire ultra-élevé ayant un poids moléculaire de 400 000 ;
(b) soumettre ledit article à une température de 190 à 340°C dans une atmosphère inerte pendant au moins 0,5 heure ;
(c) refroidir l'article sans hâte jusqu'à une température d'environ 130°C ou moins.

13. Le procédé de la revendication 12, dans lequel l'étape (a) est exécutée après l'étape (c).

14. Le procédé de la revendication 12 ou 13, dans lequel ladite température dans l'étape (b) est de 320° à 340°C.

15. Le procédé de la revendication 12, 13 ou 14, dans lequel la température dans l'étape (b) est maintenue pendant au moins 1 heure.
